(19) Europäisches Patentamt
European Patent Office
Office européen des brevets

(11) Numéro de publication: **0 025 764**
**A1**

# (12) DEMANDE DE BREVET EUROPEEN

(21) Numéro de dépôt: **80401313.4**

(22) Date de dépôt: **12.09.80**

(51) Int. Cl.[3]: **C 07 C 143/75**, C 07 C 143/74, C 07 C 7/08, C 07 C 7/10, C 10 G 21/22

(30) Priorité: **14.09.79 FR 7923040**

(43) Date de publication de la demande: **25.03.81 Bulletin 81/12**

(84) Etats contractants désignés: **AT BE DE GB IT NL**

(71) Demandeur: **COMPAGNIE FRANCAISE DE RAFFINAGE *Société anonyme dite:*, 5, rue Michel-Ange, F-75781 Paris Cedex 16 (FR)**
Demandeur: **ANVAR Agence Nationale de Valorisation de la Recherche, 43, rue Caumartin, F-75436 Paris Cedex 09 (FR)**

(72) Inventeur: **Atlani, Martial, 52, rue Bouret, F-75019 Paris (FR)**
Inventeur: **Loutaty, Roben, 29, rue Séry, F-76600 Le Havre (FR)**
Inventeur: **Wakselman, Claude, 18, rue du Clos d'Alençon, F-91120 Villebon sur Yvette (FR)**
Inventeur: **Yacono, Charles, 68, rue Armand Barbès, F-76600 Le Havre (FR)**

(74) Mandataire: **Jolly, Jean-Pierre et al, Cabinet BROT 83, rue d'Amsterdam, F-75008 Paris (FR)**

(54) **Procédé de préparation de sulfonamides aliphatiques, sulfonamides préparées par ce procédé et application de ces sulfonamides à la séparation d'hydrocarbures diéniques ou aromatiques.**

(57) Pour préparer des sulfonamides de formule

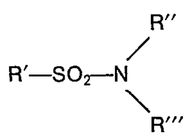
,

où R′, R″ et R‴ sont des restes alkyle, linéaires ou ramifiés, saturés ou insaturés, et possédant de 1 à 18 atomes de carbone, deux ou trois des restes R′, R″ et R‴ pouvant être identiques,
on fait réagir en phase aqueuse un halogénure d'acide sulfonique R′—$SO_2$—X avec une amine primaire

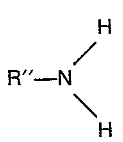
,

pour obtenir une sulfonamide

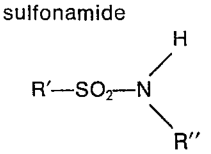
,

puis, toujours en phase aqueuse et sans séparer cette sulfonamide, on la fait réagir avec un composé contenant un reste alkyl R‴.

Les sulfonamides obtenues peuvent être appliquées à la séparation d'hydrocarbures diéniques ou aromatiques de coupes d'hydrocarbures les contenant.

EP 0 025 764 A1

**Procédé de préparation de sulfonamides aliphatiques, sulfonamides préparées par ce procédé et application de ces sulfoamides à la séparation d'hydrocarbures diéniques ou aromatiques.**

La présente invention concerne un procédé de préparation de sulfonamides aliphatiques et les sulfonamides préparées par ledit procédé.

Les sulfonamides, qui peuvent être préparées par le procédé selon la présente invention, sont des composés de formule générale :

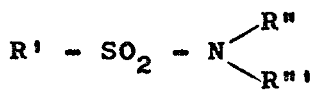

où R', R" et R"' sont des restes alkyle, linéaires ou ramifiés, saturés ou insaturés et possédant de 1 à 18 atomes de carbone.

Des procédés de préparation des sulfonamides aliphatiques sont déjà connus.

On peut citer, par exemple, la méthode indiquée par MORIARTY dans un article paru dans "The Journal of Organic Chemistry", 1965, volume 30, pages 600 et suivantes.

Cette méthode consiste à faire réagir, dans de l'éther éthylique, le chlorure d'un acide sulfonique sur une amine secondaire, suivant la réaction :

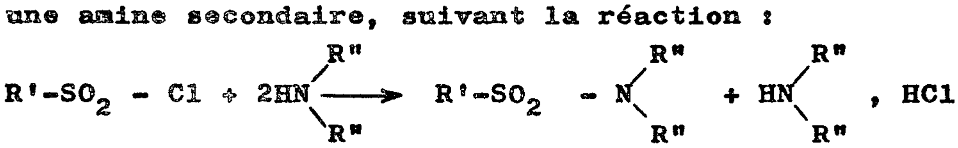

H.K. HALL Jr, dans un article paru dans "The Journal of American Chemical Society" (1956), volume 78, pages 2717 et 2718, a décrit une méthode voisine, où la réaction a lieu en présence de soude selon la réaction :

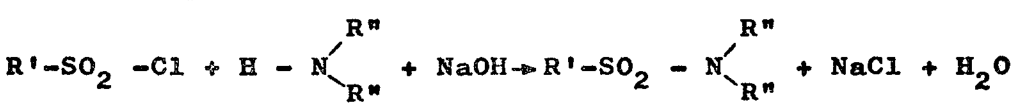

Ces deux méthodes conduisent donc, à partir d'une amine secondaire, à la formation de sulfonamides où les restes alkyle fixés sur l'azote sont identiques.

Comme décrit dans la demande de brevet français n° 77 12 357 (publiée sous le n° 2 388 874), dont les Demanderesses sont titulaires, ces sulfonamides sont de bons solvants pour la séparation d'hydrocarbures diéniques

et/ou aromatiques de coupes d'hydrocarbures les contenant. Lors de l'étude des solvants de ce type, les Demanderesses ont mis au point un nouveau procédé de préparation de sulfonamides, dont les restes alkyle fixés sur l'azote peuvent être identiques ou différents.

Le but de la présente invention est donc l'obtention de composés de la famille des sulfonamides, pouvant notamment être utilisés comme solvants d'extraction d'hydrocarbures diéniques et/ou aromatiques.

A cet effet, l'invention a pour objet un procédé de préparation de sulfonamides de formule générale :

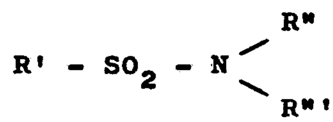

où R', R" et R"' sont des restes alkyle, linéaires ou ramifiés, saturés ou insaturés, et possédant de 1 à 18 atomes de carbone, deux ou trois des restes R', R" et R"' pouvant être identiques, ledit procédé comprenant les étapes suivantes :

a) une première étape consistant dans la réaction d'un halogénure d'acide sulfonique de formule générale $R' - SO_2 - X$ et d'une amine primaire de formule générale :

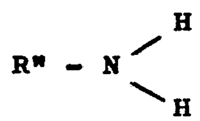

, où R' et R" sont des restes alkyle tels que définis ci-dessus et X un élément halogène choisi dans le groupe constitué par le fluor, le chlore, le brome et l'iode, ladite réaction conduisant à la formation d'une sulfonamide N monoalkyl substituée de formule générale :

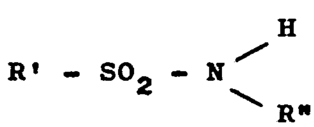

,

b) une seconde étape comprenant la réaction de la sulfonamide N monoalkyl substituée $R' - SO_2 - N<^{H}_{R''}$ avec un composé contenant au moins un reste alkyle R"' tel que défini ci-dessus, la dite seconde étape conduisant à la formation d'une sulfonamide de formule générale :

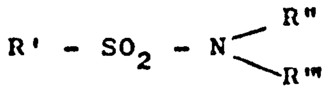

c) une troisième étape de séparation de la sulfonamide, obtenue lors de la seconde étape, du milieu réactionnel de ladite seconde étape, ledit procédé étant caractérisé en ce que la sulfonamide de formule générale

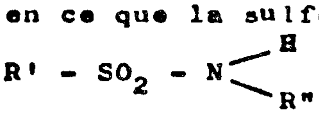 , préparée lors de la première étape,

n'est pas séparée du milieu réactionnel de ladite première étape avant la seconde étape, et en ce que la première et la seconde étapes ont lieu en phase aqueuse.

La réaction effectuée lors de la première étape s'écrit de la façon suivante:

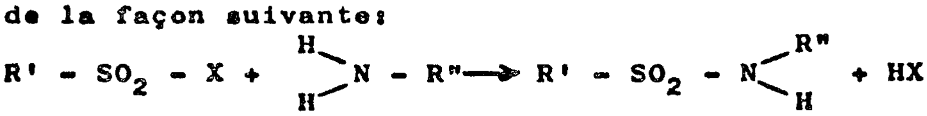 (1)

La réaction est effectuée en présence d'une solution aqueuse d'une base de formule générale B - OH, où B est un métal, par exemple alcalin, telle que la soude ou la potasse, afin de neutraliser l'acide HX formé, et donner le sel halogéné B - X.

La deuxième étape est ensuite effectuée sans séparer la sulfonamide formée du milieu réactionnel. Autrement dit, on ajoute directement à celui-ci un des composés contenant le reste alkyle R'''.

Le composé contenant le reste alkyle R''' est tel que le reste R''' se substitue à l'atome de la sulfonamide N mono alkyl substituée formée lors de la première étape. Il peut s'agir notamment:

- d'un halogénure d'alkyle R''' - X, où X est un élément halogène tel que défini ci-dessus,
- d'un sulfate d'alkyle $R'''_2 SO_4$,
- d'un sulfonate d'alkyle $R'''_2 SO_3$,
- d'un phosphate d'alkyle $R'''_3 PO_4$,
- d'un phosphonate d'alkyle $R'''_3 PO_3$.

Dans le cas d'un sulfate d'alkyle, la réaction de la deuxième étape peut s'écrire :

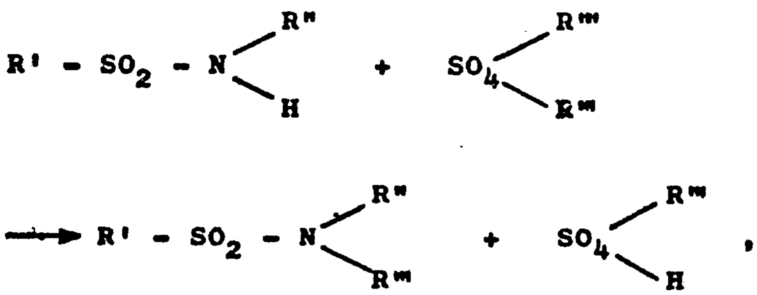

(2)

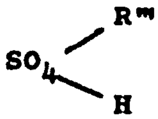 réagissant avec la base B-OH pour donner 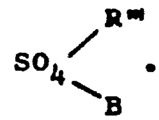.

Les Demanderesses ont établi qu'il est préférable que cette réaction soit effectuée en présence d'un excès du composé contenant le reste alkyle R‴ par rapport à la sulfonamide N monoalkyl substituée, le rendement de la réaction (2) étant alors amélioré.

Le rapport molaire $\frac{\text{Composé contenant le reste alkyle R'''}}{\text{Sulfonamide N monoalkyl substituée}}$ est ainsi, de préférence, compris entre 1 et 3.

La sulfonamide formée lors de la seconde étape est ensuite séparée du milieu réactionnel. Cette séparation peut être effectuée par une décantation de la phase organique, suivie d'une distillation de la sulfonamide.

Le procédé selon l'invention présente l'avantage, d'une part, d'être mis en oeuvre en phase aqueuse, ce qui évite l'emploi de produits de départ (comme les amines) secs et, également, de permettre la préparation de sulfonamides disubstituées dissymétriques.

Les Demanderesses ont, par le procédé selon l'invention, préparé les sulfonamides suivantes :

- la N,N, méthyl éthyl méthanesulfonamide

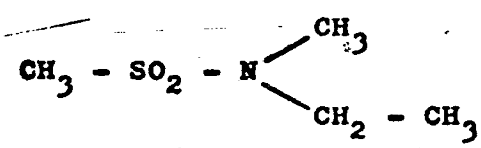

- la N,N, méthyl allyl méthanesulfonamide

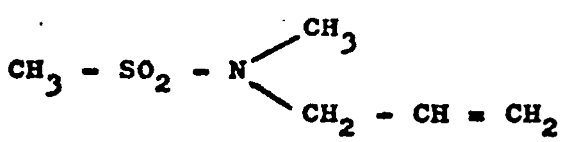

Les Demanderesses ont établi l'efficacité des sulfonamides du groupe mentionné ci-dessus dans un procédé d'extraction des hydrocarbures diéniques et/ou aromatiques à partir de coupes qui les contiennent. Cette efficacité peut être mise en évidence par plusieurs tests: on peut mesurer la sélectivité à dilution infinie du solvant considéré vis-à-vis d'un couple d'hydrocarbures témoin. On peut aussi réaliser l'extraction par une méthode classique et analyser l'extrait, d'une part, le raffinat, d'autre part. De tels tests seront rapportés ci-après dans des exemples. Quelle que soit la technique d'extraction utilisée, les quantités de solvant employées sont, en général, comprises entre 0,5 et 5 fois le volume de la charge d'hydrocarbures à traiter, mais cette quantité peut être supérieure, notamment si l'on désire réduire le nombre d'étages théoriques nécessaires à l'extraction.

Le solvant utilisé peut être plus ou moins pur: la pureté dite "commerciale" convient le plus souvent pour réaliser l'extraction. Les sulfonamides peuvent être utilisées telles quelles, en mélange entre elles ou avec d'autres sulfonamides, ou encore en mélange avec un ou plusieurs autres corps.

L'extraction peut être effectuée par extraction liquide-liquide ou par distillation extractive. Les deux techniques peuvent d'ailleurs être combinées, la charge étant enrichie, dans une première étape, en espèce chimique à extraire, par extraction liquide-liquide, puis, dans une seconde étape, soumise à une distillation extractive, qui permet de récupérer l'espèce chimique recherchée.

L'invention est illustrée par les exemples qui suivent et qui n'ont aucun caractère limitatif.

L'exemple 1 concerne la préparation de sulfonamides à l'aide du procédé selon l'invention,

Les exemples 2 et 3 concernent l'utilisation de sulfonamides préparées par le procédé selon l'invention pour l'extraction d'hydrocarbures aromatiques d'un mélange en contenant,

L'exemple 4 concerne l'utilisation de sulfonamides préparées par le procédé selon l'invention pour l'extraction d'hydrocarbures diéniques d'un mélange en contenant.

EXEMPLE 1

1. Préparation de la N,N,méthyl éthyl méthanesulfonamide

Dans un réacteur de 250 ml équipé d'un agitateur et d'un réfrigérant, on introduit 10,5 ml d'une solution aqueuse à 70% en poids d'éthylamine et une solution aqueuse de potasse contenant 10 ml d'eau et 7,5 g de potasse. Le réacteur est refroidi par un mélange de glace et de chlorure de sodium à -15°C. On introduit goutte à goutte, avec une bonne agitation, 10 ml de chlorure de méthanesulfonyle. On laisse ensuite le réacteur revenir à la température ambiante. On ajoute ensuite une solution contenant 20 ml d'eau et 18 g de potasse. On ajoute alors goutte à goutte 25 ml de sulfate de méthyle.

Il se forme une couche organique surnageante, qui est décantée. Le produit organique encore contenu dans la phase aqueuse est extrait par du chlorure de méthylène. L'extrait est mélangé à la couche organique précédemment séparée par décantation. Ce mélange est séché sur sulfate de sodium, puis le chlorure de méthylène est séparé par évaporation.

Le produit obtenu est redistillé. On obtient ainsi 10,8 g de N,N, méthyl éthyl méthanesulfonamide pure, de point d'ébullition 73°C sous 0,65 mm de mercure. Le rendement global est de 61%.

2. Préparation de la N,N, méthyl allyl méthanesulfonamide

Dans un réacteur de 250 ml équipé d'un agitateur, d'un réfrigérant et d'une ampoule à brome, on introduit 10 g d'une solution aqueuse à 40% en poids de méthylamine et une solution aqueuse de potasse contenant 10 ml d'eau et 7,5 g de potasse. Le réacteur est refroidi par un mélange de glace et de chlorure de sodium à -15°C. On introduit goutte à goutte, sous bonne agitation, 10 ml de chlorure de méthanesulfonyle. On laisse ensuite le réacteur revenir à température ambiante. On ajoute ensuite une solution contenant 20 ml d'eau et 18 g de potasse. On ajoute alors 31,5 g de bromure d'allyle.

Il se forme une couche organique surnageante, qui est décantée. Le produit organique encore contenu dans

la phase aqueuse est extrait par du chlorure de méthylène. L'extrait est mélangé à la couche organique précédemment séparée par décantation. Ce mélange est séché sur sulfate de sodium, puis le chlorure de méthylène est séparé par évaporation.

Le produit obtenu est distillé. On obtient ainsi 9,7 g de N,N, méthyl allyl méthanesulfonamide de point d'ébullition 68°C sous 0,01 mm de Hg. Le rendement global est de 50%.

EXEMPLE 2

Cet exemple illustre l'utilisation de solvants selon l'invention, pour l'extraction d'hydrocarbures aromatiques de mélanges d'hydrocarbures les contenant, par distillation extractive, ou par extraction liquide-liquide, ou par une combinaison de ces deux techniques.

On a déterminé, pour deux solvants selon l'invention, à savoir la N,N, méthyl allyl méthanesulfonamide et la N,N, méthyl éthyl méthanesulfonamide, la sélectivité à dilution infinie, à 50°C, vis-à-vis de deux couples d'hydrocarbures ayant des points d'ébullition voisins: le couple benzène - 2,4 diméthylpentane et le couple benzène-cyclohexane.

La sélectivité à dilution infinie d'un solvant, vis-à-vis d'un couple d'hydrocarbures que l'on se propose de séparer par distillation extractive ou par extraction liquide-liquide, se définit comme le rapport des coefficients d'activité à dilution infinie des deux hydrocarbures dans ce même solvant. La séparation est d'autant plus efficace que la sélectivité à dilution infinie est plus élevée (voir "Propriétés thermodynamiques des solutions infiniment diluées d'hydrocarbures dans les solvants polaires" par P. VERNIER, C. RAIMBAULT et H. RENON - Journal de Chimie Physique, 1969, V. 66, n° 3, pages 429 à 436).

Les coefficients d'activité à dilution infinie ont été mesurés par la méthode du "diluteur exponentiel" (voir "Accurate measurement of activity coefficients at infinite dilution by inert gas stripping and gas chromato-

graphy" par J-C LERDI, J-C MASSON, H. RENON, J-F FABRIES et H. SANNIER, Ind. Eng. Chem. Process Des. Dev., V. 16, n° 1, pages 139 à 144, 1977).

Les résultats sont donnés dans le tableau 1 ci-après.

**TABLEAU 1**

| Hydrocarbures | | Coefficients d'activité à dilution infinie à 50°C | | Sélectivités à dilution infinie à 50°C par rapport au benzène | |
|---|---|---|---|---|---|
| Nom | Température d'ébullition sous 760 mm Hg (°C) | dans la N,N, méthyl allyl méthanesulfonamide | dans la N,N, méthyl éthyl méthanesulfonamide | dans la N,N, méthyl allyl méthanesulfonamide | dans la N,N, méthyl éthyl méthanesulfonamide |
| Benzène | 80,1 | 1,37 | 1,58 | 1 | 1 |
| Cyclohexane | 80,7 | 6,76 | 10,11 | 4,93 | 6,40 |
| 2,4,diméthylpentane | 80,5 | 9,85 | 21,06 | 7,19 | 13,33 |

Les valeurs obtenues pour les sélectivités à dilution infinie montrent que les séparations sont efficaces. On peut donc extraire le benzène de ses mélanges avec le diméthyl-2,4 pentane ou avec le cyclohexane en utilisant les solvants selon l'invention.

EXEMPLE 3

Cet exemple concerne l'utilisation de solvants selon l'invention pour l'extraction d'un hydrocarbure aromatique par extraction liquide-liquide.

On prépare dans une ampoule à décanter des mélanges ternaires de benzène (ci-après dénommé le soluté), de n-heptane (ci-après dénommé le diluant) et d'un solvant selon l'invention.

Après agitation, on laisse reposer à la température de 20°C. On obtient ainsi deux phases.

Les résultats des essais effectués figurent dans le Tableau 2 ci-après, où :

- le coefficient de partage est le rapport :

$$\frac{\text{fraction massique HC (soluté ou diluant ) dans l'extrait}}{\text{fraction massique du même HC dans le raffinat}},$$

HC signifiant hydrocarbure,

- le facteur de séparation est le rapport :

$$\frac{\text{coefficient de partage du soluté}}{\text{coefficient de partage du diluant}}$$

Les fractions massiques des mélanges ternaires ont été déterminées par pesée, celles de l'extrait et du raffinat par chromatographie en phase gazeuse.

TABLEAU 2

| Mélange ternaire à 20°C | Composition du mélange en % en poids | Composition de l'extrait en % en poids | Composition du raffinat en % en poids | Coefficients de pourcentage | Facteur de séparation |
|---|---|---|---|---|---|
| Benzène | 17,60 | 16,26 | 20,91 | 0,778 | 8,46 |
| n-heptane | 32,80 | 6,94 | 75,59 | 0,092 | |
| N,N-méthyl allyl méthane-sulfonamide | 49,60 | 76,80 | 3,50 | | |
| Benzène | 17,92 | 15,53 | 21,14 | 0,735 | 11,48 |
| n-heptane | 32,37 | 4,75 | 74,16 | 0,064 | |
| N,N-méthyl éthyl méthane-sulfonamide | 49,71 | 79,72 | 4,70 | | |

Les valeurs obtenues pour les facteurs de séparation montrent que les séparations sont efficaces. On peut donc extraire le benzène de son mélange avec le n-heptane à l'aide des solvants selon l'invention.

EXEMPLE 4

Cet exemple concerne l'utilisation de solvants selon l'invention pour l'extraction d'hydrocarbures diéniques de mélanges d'hydrocarbures les contenant, par distillation extractive, ou par extraction liquide-liquide, ou par une combinaison de ces deux techniques.

On a déterminé pour deux solvants selon l'invention, la sélectivité à dilution infinie à 30°C vis-à-vis de deux couples d'hydrocarbures ayant des points d'ébullition voisins : le couple isoprène - n-pentane et le couple isoprène - méthyl-2 butène-2.

La sélectivité à dilution infinie est définie et déterminée de la même façon que pour l'exemple 2.

Les résultats sont donnés dans le Tableau 3.

TABLEAU 3

| Hydrocarbures | | Coefficients d'activité à dilution infinie à 30°C | | Sélectivités à dilution infinie à 30°C par rapport à l'isoprène | |
|---|---|---|---|---|---|
| Nom | Température d'ébullition sous 760 mm Hg (°C) | dans la N,N méthyl allyl méthanesulfonamide | dans la N,N méthyl éthyl méthanesulfonamide | dans la N,N méthyl allyl méthanesulfonamide | dans la N,N méthyl éthyl méthanesulfonamide |
| Isoprène | 34,1 | 3,03 | 3,55 | 1 | 1 |
| Méthyl-2-butène-2 | 38,6 | 5,69 | 7,28 | 1,88 | 2,05 |
| n-pentane | 36,1 | 11,88 | 15,16 | 3,92 | 4,27 |

Les valeurs obtenues pour les sélectivités à dilution infinie montrent que les séparations sont efficaces. On peut donc extraire l'isoprène de ses mélanges avec le n-pentane ou avec le méthyl-2-butène-2 en utilisant les solvants selon l'invention.

REVENDICATIONS

1.- Procédé de préparation de sulfonamides de formule générale:

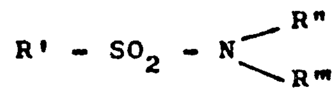

où R', R" et R‴ sont des restes alkyle, linéaires ou ramifiés, saturés ou insaturés, et possédant de 1 à 18 atomes de carbone, deux ou trois des restes R', R" et R‴ pouvant être identiques, ledit procédé comprenant les étapes suivantes :

a) une première étape consistant dans la réaction, d'un halogénure d'acide sulfonique de formule générale R' - $SO_2$ - X, et d'une amine primaire de formule générale

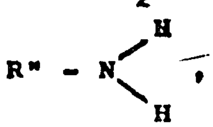, où R' et R" sont des restes alkyle tels que définis ci-dessus et X un élément halogène choisi dans le groupe constitué par le fluor, le chlore, le brome et l'iode, ladite réaction conduisant à la formation d'une sulfonamide N monoalkyl substituée de formule générale:

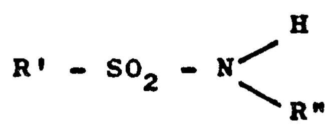

b) une seconde étape comprenant la réaction de la sulfonamide N monoalkyl sulbstituée 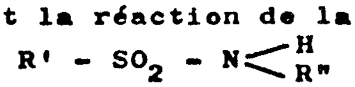 avec un composé contenant au moins un reste alkyle R‴ tel que défini ci-dessus,

ladite seconde étape conduisant à la formation de la sulfonamide de formule générale

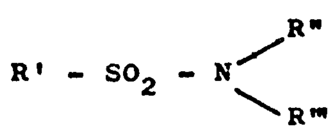

c) une toisième étape de séparation de la sulfonamide obtenue lors de la seconde étape, du milieu réactionnel de ladite seconde étape,

ledit procédé étant caractérisé en ce que la sulfonamine de formule générale:

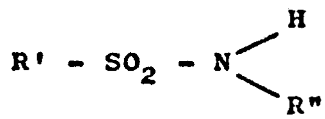

préparée lors de la première étape, n'est pas séparée du milieu réactionnel de ladite première étape avant la seconde étape, et en ce que la première et la seconde étapes ont lieu en phase aqueuse.

2.- Procédé selon la revendication 1, caractérisé en ce que le composé contenant le reste R''' est choisi dans le groupe constitué par

- les halogénures d'alkyle $R'''X$,
- les sulfates d'alkyle $R'''_2 SO_4$,
- les sulfonates d'alkyle $R'''_2 SO_3$,
- les phosphonates d'alkyle $R'''_3 PO_3$ et
- les phosphates d'alkyle $R'''_3 PO_4$.

3.- Procédé selon l'une des revendications 1 et 2, caractérisé en ce que la réaction de la deuxième étape est effectuée en présence d'un excès du composé contenant le reste R'''.

4.- Procédé selon la revendication 2, caractérisé en ce que, lors de la réaction de la deuxième étape, le rapport molaire $\frac{\text{Composé contenant le reste R'''}}{\text{Sulfonamide N mono alkyl substituée}}$ est compris entre 1 et 3.

5.- Sulfonamides préparées par le procédé selon les revendications 1 à 4.

6.- Sulfonamide selon la revendication 5, caractérisée en ce que ladite sulfonamide est la N,N, méthyl éthyl méthanesulfonamide.

7.- Sulfonamide selon la revendication 5, caractérisée en ce que ladite sulfonamide est la N,N, méthyl allyl méthanesulfonamide.

8.- Application des sulfonamides selon l'une des revendications 5 à 7, à la séparation d'hydrocarbures diéniques et/ou aromatiques de coupes d'hydrocarbures les contenant.

0025764

Office européen des brevets

## RAPPORT DE RECHERCHE EUROPEENNE

Numéro de la demande

EP 80 40 1313

### DOCUMENTS CONSIDERES COMME PERTINENTS

| Catégorie | Citation du document avec indication, en cas de besoin, des parties pertinentes | Revendication concernée |
|---|---|---|
| | GB - A - 818 756 (MINNESOTA MINING & MANUFACTURING CO.)<br>* Page 1, lignes 63-69; exemple 1 *<br>-- | 1-8 |
| D | FR - A - 2 388 874 (CIE. FRANCAISE DE RAFFINAGE ET ANVAR)<br>* Page 2, ligne 5 - page 4, ligne 10; revendications *<br>-- | 1-8 |
| | JOURNAL OF THE AMERICAN CHEMICAL SOCIETY, vol. 80, mai 1958, Washington D.C., US<br>C.W. WHITEHEAD et al. "Diuretics. II. Alkoxymercuration by mixed anion salts of mercury", pages 2182-5.<br>* Page 2184, table I; page 2183, colonne de droite: N-allylakane-sulfonamides" *<br>-- | 1-8 |
| | JOURNAL OF THE AMERICAN CHEMICAL SOCIETY, vol. 97, avril 1975 Washington D.C., US<br>J.F. KING et al. "Quaternary methylsulfonylammonium salts. New reagents for mesylation. The mechanism of hydrogen multi-exchange in sulfene reactions", pages 2566-7.<br>* Page 2566, formule 5C *<br>---- | 1-8 |

CLASSEMENT DE LA DEMANDE (Int. Cl. 3)

C 07 C 143/75
143/74
7/08
7/10
C 10 G 21/22

DOMAINES TECHNIQUES RECHERCHES (Int. Cl. 3)

C 07 C 143/74
143/75
7/10
7/08
C 10 G 21/22

CATEGORIE DES DOCUMENTS CITES

- X: particulièrement pertinent
- A: arrière-plan technologique
- O: divulgation non-écrite
- P: document intercalaire
- T: théorie ou principe à la base de l'invention
- E: demande faisant interférence
- D: document cité dans la demande
- L: document cité pour d'autres raisons
- &: membre de la même famille, document correspondant

✓ Le présent rapport de recherche a été établi pour toutes les revendications

| Lieu de la recherche | Date d'achèvement de la recherche | Examinateur |
|---|---|---|
| La Haye | 10-12-1980 | PAUWELS |

OEB Form 1503.1 06.78